# EUROPEAN PATENT SPECIFICATION

(11) **EP 4 134 111 B1**
(45) Date of publication and mention of the grant of the patent: **17.12.2025**
(21) Application number: 21870478.1
(22) Date of filing: 02.07.2021
(51) Int. Cl.: A61M 5/20

(54) **AUTOINJECTOR AND ACTUATING ASSEMBLY THEREOF**
AUTOINJEKTOR UND SEINE BETÄTIGUNGSANORDNUNG
AUTO-INJECTEUR ET SON ENSEMBLE D'ACTIONNEMENT

(43) Date of publication of application: 15.02.2023
(73) Proprietor: Zhejiang Summed Medtech Co., Ltd., Huzhou, Zhejiang 313000 (CN)
(72) Inventor: XIE, Yingqiang, Shanghai 200000 (CN)
(74) Representative: Schwerbrock, Florian
(86) International application number: PCT/CN2021/104229
(87) International publication number: WO 2023/272716

(56) References cited:
- WO-A1-2010/115670
- WO-A1-2012/173553
- WO-A1-2019/224785
- CN-A- 102 497 899
- CN-A- 105 246 528
- CN-A- 106 061 529
- CN-A- 112 165 963
- CN-A- 112 933 346
- US-A1- 2017 337 845
- US-A1- 2019 167 903
- US-B1- 10 357 619

## Description

### FIELD OF DISCLOSURE

The present invention relates to an actuation assembly of an auto-injector, which is capable of actuating a mounted vial to perform an injection in particular. The present invention also relates to an auto-injector using the above-described actuation assembly.

### BACKGROUND OF THE INVENTION

Previously, when a patient needed an injection, he or she always needed a medical staff to assist the injection procedure. With the advancement of medical technology, auto-injector has been widely used because it can be operated and injected by patients themselves. What patients need to do is operating the automatic injection needle with one hand to complete the injection, which saves medical manpower and resources and improves the convenience and flexibility of the injection.

However, in order to operate more easily and perform automatic injection, the existing auto-injector is often too complicated in structure design, which makes such injector unfavorable for assembly and increases the manufacturing cost. Therefore, how to simplify the structure and quantity of the assembled parts, reduce the assembly difficulty of the auto-injector, and make it easier for the user to operate, is a topic worthy of research. Prior art auto-injectors are disclosed in WO2010/115670 A1, WO2012/173553 A1 and WO2019/224785 A1.

### SUMMARY OF INVENTION

The present invention is made in view of the above issues, with the purpose of providing an auto-injector and its actuation assembly, which can simplify the structural design, improve the convenience of assembly, and meet the medical regulations and standards of the assembly and injection process.

The actuation assembly of an auto-injector provided in the invention comprises an actuation sleeve, an execution sleeve, a first elastic element, a rear cover, a push rod, and a second elastic element. The actuation sleeve includes a channel. The execution sleeve, which is movably threaded through the channel along an axis, the execution sleeve includes two symmetrical cantilevers, and each cantilever arm includes a clamping part. Each end of the first elastic element abuts the actuation sleeve and the execution sleeve, respectively. The rear cover is coupled to one end of the execution sleeve. The push rod, which is movably threaded through the execution sleeve along the axis, and the push rod includes two grooves. Each end of the second elastic element abuts against the rear cover and the push rod, respectively. Wherein, when the cantilever arm of the execution sleeve is confined within the channel of the actuation sleeve, the clamping part of the cantilever arm is clipped into the groove of the push rod to restrict the movement of the push rod; when the actuation sleeve moves along the axis direction relative to the execution sleeve, the first elastic element is gradually compressed, so that the clamping part of the cantilever arm of the execution sleeve is disengaged from the groove of the push rod, and the push rod is actuated toward the vial by the second elastic element which has been compressed.

In one embodiment of the present invention, the actuation sleeve includes a protruding part, which is disposed on the outer surface of the actuation sleeve, and the execution sleeve further includes a plurality of abutting portions. Both ends of the first elastic element abuts the protruding part of the actuation sleeve and the plurality of abutting portions of the execution sleeve, respectively.

In an embodiment of the present invention, the actuation sleeve further comprises a plurality of grooves, and the plurality of abutting portions of the actuation sleeve pass through the grooves to protrude from the outer surface of the actuation sleeve. And, when the actuation sleeve moves relative to the actuation sleeve, the abutting portions move along the grooves.

In one embodiment of the present invention, the execution sleeve further comprises a plurality of buckle parts, and the rear cover further comprises a plurality of buckle elements corresponding to the buckle parts.

In one embodiment of the present invention, the rear cover further comprises a plurality of guide elements, and the execution sleeve further includes a plurality of corresponding guide elements; when the rear cover moves relative to the execution sleeve, the rear cover is moved relative to the execution sleeve along the axis with the cooperation between the guide elements and the corresponding guide elements.

In one embodiment of the present invention, the actuation sleeve further comprises at least one stopper structure, which is protruded on the surface of the channel.

In one embodiment of the present invention, each of the cantilevers further comprises an outer expansion part corresponding to the stopper structure.

In one embodiment of the present invention, the actuation sleeve further comprises at least one auxiliary mounting hole, which passes through the outer surface of the actuation sleeve to the channel, and is located between one end of the actuation sleeve adjacent to the rear cover and at least one stopper structure.

The auto-injector of the present invention includes the aforementioned actuation assembly and accommodating assembly. The accommodating assembly is coupled to one end of the actuation assembly and includes an outer housing, a vial sleeve and a needle cap. A vial sleeve coupled to the outer housing for mounting the vial; A needle cap is threaded through and partially exposed to the outer housing and can move relative to the outer housing and the vial sleeve. Wherein, when the needle cap is moved toward the outer housing by an external force, the needle cap pushes against the actuation sleeve of the actuation assembly, causing the clamping part of the cantilever arms of the actuation sleeve to disengage the groove of the push rod, which in turn actuates the push rod through the second elastic element which has been compressed, thereby actuating the push rod to move toward the vial to complete the injection.

In one embodiment of the present invention, the auto-injector further comprises a backing ring, which is disposed at one end of the actuating component adjacent to the vial sleeve.

The auto-injector and its actuation assembly in the present invention can simplify the structural design and improve the convenience of assembly by implementing the structural design of the two symmetrical cantilevers of the execution sleeve and the corresponding parts. Besides, it meets the medical regulations and standards of the assembly and injection process by providing an audible sound to alert the assembly in place and the end of automatic injection.

The specific technology adopted in the present invention will be further described by the following examples and accompanying drawings.

### BRIEF DESCRIPTION OF THE DRAWINGS

FIG.1 is an exploded view of the actuation assembly of the auto-injector in the present invention.
FIG.2 is a schematic diagram of the actuation assembly of the auto-injector of the present invention.
FIG.3 is a cross-sectional view of the actuation sleeve of the actuation assembly of the auto-injector in the present invention threaded through the execution sleeve.
FIG.4 is an exploded view of the auto-injector in the present invention.
FIG.5 is a schematic view of the auto-injector in the present invention.
FIG.6 is an exploded view of the accommodating assembly of the auto-injector in the present invention.

### Symbol description of main components:

1 Auto-Injector
10 Actuation assembly
11 Actuation sleeve
111 Channel
112 Protruding part
113 Groove
114 Joint part
115 Auxiliary mounting hole
116 Stopper structure
12 Execution sleeve
121 Cantilever arm
1211 Clamping part
1212 Outer expansion part
122 Abutting portion
123 Buckle part
124 Guide element
13 First elastic element
14 Rear cover
141 Buckle element
142 Corresponding guide element
143 Locating element
15 Push rod
151 Groove
16 Second elastic element
20 Accommodating assembly
21 Outer housing
211 Buckle hole
212 Inspection hole
22 Vial sleeve
221 Inspection part
23 Needle cap
231 Sliding groove
232 Corresponding joint part
24 Backing ring
30 Vial
L Axis

### DETAILED DESCRIPTION

In order to fully understand the object, characteristics and effects of the present invention, the present invention is described in detail as below by the following specific embodiments and the accompanying drawings.

In the present invention, the use of "a" or "an" is used to describe the unit, element and component described herein. This is done only for convenience of description and to provide a general sense of the scope of the invention. Thus, unless obviously it is meant otherwise, such descriptions should be read to include a or an, at least a or an, and the singular also includes the plural.

In the present invention, the terms "comprising", "including", "having", "containing" or any other similar terms are intended to cover non-exclusive inclusions. For example, an element, structure, article, or device that contains multiple elements is not limited to those elements listed herein, but can include those elements that are not explicitly listed but are generally inherent to the element, structure, article, or device. Otherwise, unless expressly stated to the contrary, the term "or" refers to an inclusive "or", rather than an exclusive "or".

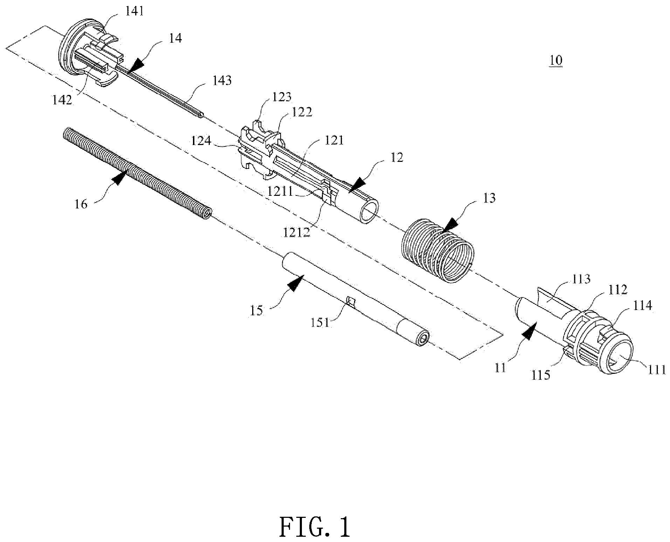

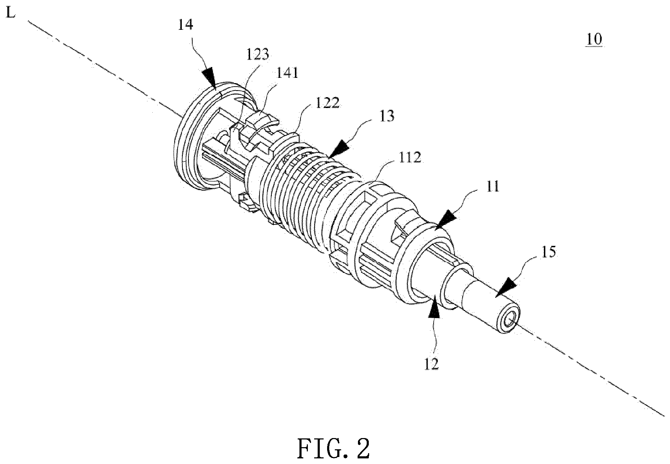

The actuation assembly of the auto-injector of the present invention is used in combination with an accommodating assembly mounted with a vial, and the vial is actuated by the actuation assembly to perform an injection operation towards users. Please refer to FIG.1 and FIG.2 first, wherein FIG.1 is an exploded view of the actuation assembly of the auto-injector of the present invention, and FIG.2 is a schematic diagram of the actuation assembly of the auto-injector of the present invention. As shown in FIG.1 and 2, the actuation assembly 10 of the auto-injector of the present invention comprises an actuation sleeve 11, an execution sleeve 12, a first elastic element 13, a rear cover 14, a push rod 15 and a second elastic element 16. The actuation assembly 10 as a whole corresponds to one axis L, so that the movable parts of the actuation assembly 10 move relative to other parts along the direction of said axis L. The actuation sleeve 11, the execution sleeve 12, the rear cover 14 and the push rod 15 can be made of the same or different solid materials, such as plastic material, metal or alloy, etc., while the first elastic element 13 and the second elastic element 16 can be made of metal or alloy. In structural design, the actuation sleeve 11, the execution sleeve 12 and the push rod 15 are all similar in appearance to tubular parts, so that the hollow parts of the aforementioned parts can accommodate corresponding parts, but the invention is not limited thereto.

The actuation sleeve 11 includes a channel 111 and a protruding part 112. The channel 111 goes through one end of the actuation sleeve 11 to the other end, so as to thread through the components such as the actuation sleeve 12. The protruding part 112 is disposed on the outer surface of the actuation sleeve 11, and the protruding part 112 protrudes from the outer surface to a sufficient height for abutting against the first elastic element 13.

The actuation sleeve 11 also includes a plurality of grooves 113 and a plurality of joints 114. Each groove 113 is a semi-open groove. That is, each groove 113 extends from one end of the actuation sleeve 11 to the other end but does not reach the other end. Wherein, the plurality of grooves 113 are two grooves arranged symmetrically, but the number and position of the plurality of grooves 113 are not limited thereto. A plurality of joint parts 114 are disposed at the actuation sleeve 11 to form opposite ends of the plurality of grooves 113 for interlocking with the aforementioned accommodating assembly. Wherein, the plurality of joint parts 114 are two symmetrically arranged bumps, but the number and position of the plurality of joint parts 114 are not limited thereto.

In one embodiment of the present invention, the actuation sleeve 11 further comprises at least one auxiliary mounting hole 115, which threads through the outer surface of the actuation sleeve 11 to the channel 111. At least one auxiliary mounting hole 115 provides a path for an external tool to assist in threading through the execution sleeve 12. Wherein, at least one auxiliary installation hole 115 is two symmetrically arranged openings, but the number and position of the at least one auxiliary installation hole 115 are not limited thereto.

The execution sleeve 12 is movably threaded through the channel 111 of the actuation sleeve 11 along the axis L. The execution sleeve 12 includes two symmetrical cantilever arms 121, which control the movement of the push rod before and after the actuation sleeve 11 is actuated. Each cantilever arms 121 basically extends along axis L, and one end of each cantilever arms 121 is connected to the main body of the execution sleeve 12 to form a fulcrum, and the other end is a suspended end, enabling it to swing based on the fulcrum and change the distance from the axis L. Each cantilever arms 121 includes a clamping part 1211 and an outer expansion part 1212 disposed at the aforementioned suspended ends, wherein the clamping part 1211 extends in a direction close to the axis L, and the outer expansion part 1212 extends in a direction away from the axis L and the parallel axis L, such that the surrounding area of the actuation sleeve 12 based on the direction of the vertical axis L at the location of the clamping portion 1211 has a minimum cross-sectional diameter length of the actuation sleeve 12, and the surrounding area based on the direction of the vertical axis L at the location of the outer expansion part 1212 has the maximum cross-sectional diameter length of the actuation sleeve 12. Wherein, the clamping part 1211 is a protruding block, and the outer expansion part 1212 forms a structure with a pointed shape along the axis L.

The execution sleeve 12 also includes a plurality of abutting portions 122. A plurality of abutting portions 122 are disposed on the outer surface of the actuation sleeve 12, and a plurality of abutting portions 122 protrude from the outer surface at a sufficient height to abut the first elastic element 13. In an embodiment of the present invention, when the execution sleeve 12 is threaded through the channel 111 of the actuation sleeve 11, and the plurality of abutting portions 122 of the actuation sleeve 12 pass through a plurality of grooves 113 to protrude from the outer surface of the actuation sleeve 11. And when the actuation sleeve 12 moves relative to the actuation sleeve 11, the plurality of abutting portions 122 move along the plurality of grooves 113.

In one embodiment of the present invention, the execution sleeve 12 also includes a plurality of buckle parts 123. A plurality of buckle parts 123 is disposed at one end of the rear cover 14 for buckling the rear cover 14. Wherein, the plurality of buckle parts 123 are symmetrically arranged blocks, and each stopper forms a slope structure on the side facing the rear cover 14, but the number and position of the plurality of buckle parts 123 are not limited thereto. A plurality of joint parts 114 are disposed at the actuation sleeve 11 to form opposite ends of the plurality of grooves 113 for interlocking with the aforementioned accommodating assembly. Wherein, the plurality of joint parts 114 are two symmetrically arranged blockers, but the number and position of the plurality of joint parts 114 are not limited thereto.

The execution sleeve 12 also includes a plurality of guide elements 124. A plurality of guide elements 124 are disposed at one end of the execution sleeve 12 coupled to the rear cover 14. Wherein, the plurality of guide elements 124 are two symmetrically arranged semi-open grooves, and each groove extends from the end of the execution sleeve 12 in the direction parallel to the axis L, but the number and location of the plurality of guide elements 124 are not limited thereto.

Two ends of the first elastic element 13 abut against the actuation sleeve 11 and the execution sleeve 12 respectively. In one embodiment of the present invention, both ends of the first elastic element 13 abut against the protruding part 112 of the actuation sleeve 11 and the plurality of abutting portions 122 of the execution sleeve 12 respectively, and the cross-sectional diameter of the first elastic element 13 is greater than the minimum cross-sectional diameter of the outer surfaces of the actuation sleeve 11 and the execution sleeve 12, so that the first elastic element 13 is roughly assembled on the outer surfaces of the actuation sleeve 11 and the execution sleeve 12. As the actuation sleeve 11 moves in the direction of the axis L relative to the execution sleeve 12, the first elastic element 13 is compressed as the protruding part 112 of the actuation sleeve 11 approaches the plurality of abutting portions 122 of the execution sleeve 12. On the contrary, as the protruding part 112 of the actuation sleeve 11 moves away from the plurality of abutting portions 122 of the execution sleeve 12, the first elastic element 13 is expanded by elastic restoring force.

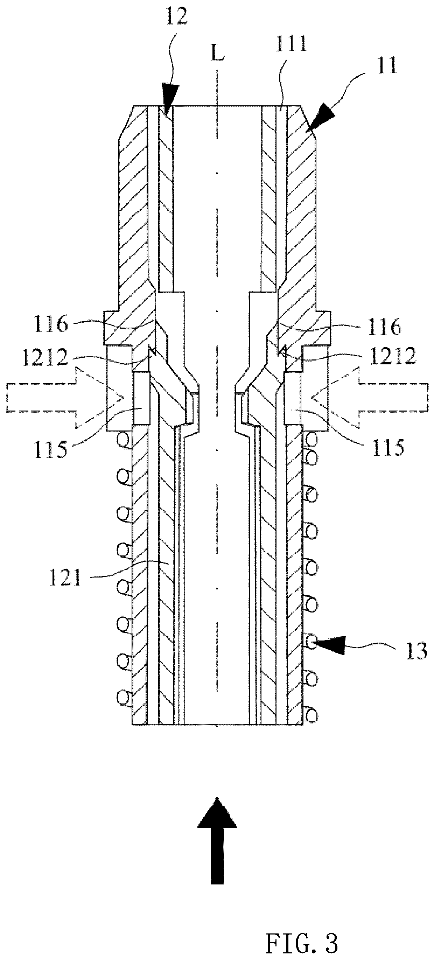

Please refer to FIG.3, which is a cross-sectional view of the actuation sleeve of the actuation assembly of the auto-injector of the present invention threaded through the execution sleeve. As shown in FIG. 3, in one embodiment of the present invention, the actuation sleeve 11 of the actuation assembly 10 of the auto-injector of the present invention further includes at least one stopper structure 116. At least one blocking structure 116 is protruded from the surface of the channel 111. Wherein, at least one stopper structure 116 is an annular convex structure. And the annular convex structure and the surface of the channel 111 of the actuation sleeve 11 form a recessed space between them that corresponds to the direction of penetration of the actuation sleeve 12 and into which the tip of the outer expansion part 1212 can be seated. In terms of structural design, the cross-sectional diameter of the channel 111 of the actuation sleeve 11 based on the direction of the vertical axis L is smaller than the maximum cross-sectional diameter of the execution sleeve 12 (For example, the cross-sectional diameter corresponding to the outer expansion part 1212 of the two cantilevers 121 of the execution sleeve 12). Accordingly, when the execution sleeve 12 is threaded along the axis L direction relative to the actuation sleeve 11, the execution sleeve 12 cannot directly pass through the channel 111 of the actuation sleeve 11. At this time, the outer expansion part 1212 of the second cantilever arms 121 of the actuation sleeve 12 must first be pressed to swing towards the axis L, so that the maximum cross-sectional diameter of the actuation sleeve 12 is not greater than the cross-sectional diameter of the channel 111 of the actuation sleeve 11 based on the direction of the vertical axis L. The threading operation is then performed. Next, when the outer expansion part 1212 of the cantilever arms 121 of the actuation sleeve 12 reaches the position of the at least one stopper structure 116, the tip of the outer expansion part 1212 will snap into the recessed space formed between the surfaces of at least one stopper structure 116 and the channel 111 of the actuation sleeve 11 and thus restrict the movement of the execution sleeve 12. At this time, the assembler must use an external tool to pass through the aforementioned at least one auxiliary installation hole 115 and press the cantilever arms 121 of the execution sleeve 12, so that the cantilever arms 121 of the execution sleeve 12 moves toward the direction close to the axis L to reduce the maximum cross-sectional diameter of execution sleeve 12. In this way, the execution sleeve 12 can pass through the at least one stopper structure 116 and continue to complete the threading operation. During the aforementioned process, the tip of the outer expansion part 1212 of the cantilever arms 121 will rattle as it bounces off at least one stop structure 116 by an external force, so that the assembler can confirm whether the tip of the outer expansion part 1212 of the cantilever arms 121 has disengaged from the recessed space.

As shown in FIG.1 and FIG.2, the rear cover 14 is combined with one end of the execution sleeve 12. In an embodiment of the present invention, the rear cover 14 includes a plurality of buckle elements 141, and the plurality of buckle elements 141 are correspondingly fastened to the plurality of buckle parts 123 of the execution sleeve 12. Wherein, the plurality of buckle elements 141 are a plurality of elastic arm structures arranged symmetrically, and each elastic arm structure extends toward the execution sleeve 12 along the direction parallel to the axis L. And a spring sheet extending in the direction of the vertical axis L is produced on the side close to the execution sleeve 12. The number, position and structure of the plurality of buckle elements 141 are matched to the plurality of buckle parts 123 of the aforementioned execution sleeve 12. When the rear cover 14 is coupled to the execution sleeve 12, the spring sheets of the plurality of buckle elements 141 will move along the inclined surface structure of the plurality of buckle parts 123 until the spring sheets of the buckle elements 141 move to the rear of a plurality of buckle parts 123. In the aforementioned process, the spring sheets of a plurality of buckle elements 141 will make a sound due to the elastic force generated by passing over the plurality of buckle parts 123 to hit the execution sleeve 12, so that the assembler can confirm the fastening between the plurality of buckle elements 141 of the rear cover 14 and the plurality of buckle parts 123 of the execution sleeve 12. At this time, the spring sheets of the plurality of buckle elements 141 of the rear cover 14 will be blocked by the plurality of buckle parts 123 of the execution sleeve 12 along the axis L direction, so that the rear cover 14 cannot be separated from the execution sleeve 12 along the direction of the axis L.

The rear cover 14 also includes a plurality of corresponding guide elements 142. Wherein, the plurality of corresponding guide elements 142 are two ridges arranged symmetrically, and each ridge extends toward the execution sleeve 12 along the direction parallel to the axis L. The number, position and structure of the plurality of corresponding guide elements 142 are matched to the plurality of guide elements 124 of the execution sleeve 12. When the rear cover 14 is coupled to the execution sleeve 12, the plurality of corresponding guide elements 142 of the rear cover 14 are matched with the plurality of guide elements 124 of the sleeve 12, so that the rear cover 14 and the execution sleeve 12 can be combined along the direction of the parallel axis L.

In addition, in one embodiment of the present invention, the rear cover 14 further includes a locating element 143 for the second elastic element 16 to thread through and locate. Wherein, the locating element 143 is a single rod-shaped component, which extends toward the execution sleeve 12 along the direction parallel to the axis L.

The push rod 15 is mainly used to push the plunger of the vial in the accommodating assembly. The push rod 15 is movably threaded through the execution sleeve 12 along the axis L. In the embodiment of the present invention, the push rod 15 includes two grooves 151, the positions and structures of which are matched to the clamping part 1211 of the two cantilevers 121 of the aforementioned execution sleeve 12. The inside of the push rod 15 is provided with a channel running through both ends of the push rod 15, and openings of different sizes are formed at the two ends of the push rod 15. A small opening through which the second elastic element 16 cannot pass is formed at one end of the push rod 15 away from the rear cover 14.

Two ends of the second elastic element 16 abut against the rear cover 14 and the push rod 15 respectively. In one embodiment of the present invention, one end of the second elastic element 16 threads through the locating element 143 of the rear cover 14 to abut against the rear cover 14, while its other end threads through one end of the push rod 15 and abuts against the other end of the push rod 15 away from the rear cover 14. The cross-sectional diameter of the second elastic element 16 is greater than that of the locating element 143 of the rear cover 14, but smaller than that of the channel in the push rod 15. When the actuation assembly 10 of the auto-injector of the present invention is assembled, the execution sleeve 12 restricts the movement of the push rod 15, so that the second elastic element 16 is in a compressed state. When the execution sleeve 12 is actuated by the actuation sleeve 11 to remove the movement restriction for the push rod 15, the push rod 15 will be pushed against the push rod 15 to move by the elastic restoring force of the second elastic element 16.

As the actuation sleeve 11 moves in the direction of the axis L relative to the execution sleeve 12, the first elastic element 13 is compressed as the protruding part 112 of the actuation sleeve 11 approaches the plurality of abutting portions 122 of the execution sleeve 12. On the contrary, as the protruding part 112 of the actuation sleeve 11 moves away from the plurality of abutting portions 122 of the execution sleeve 12, the first elastic element 13 is expanded by elastic restoring force.

During the assembly process of the actuation assembly 10 of the auto-injector of the present invention, the execution sleeve 12 is firstly threaded through the channel 111 of the actuation sleeve 11, and the first elastic element 13 is clamped to the execution sleeve 12 and the actuation sleeve 11 at this time. Then, the rear cover 14 is combined with one end of the execution sleeve 12. After the above procedure is completed, the actuation sleeve 11 moves relative to the execution sleeve 12 along the axis L direction to compress the first elastic element 13, so that the two cantilevers 121 of the execution sleeve 12 pass through the channel 111 and are exposed to one end of the actuation sleeve 11 away from the rear cover 14.Then the push rod 15 and the second elastic element 16 are threaded through the execution sleeve 12, so that the second elastic element 16 is in a compressed state. Finally, the actuation sleeve 11 is moved in the opposite direction along the axis L direction relative to the execution sleeve 12 to the original position where the first elastic element 13 is not compressed. At this time, the two cantilever arms 121 of the execution sleeve 12 will be restricted to the channel 111 of the actuation sleeve 11 and the clamping part 1211 of each cantilever arms 121 is correspondingly clamped into the groove 151 of the push rod 15, thereby restricting the movement of the push rod 15. Accordingly, the assembly is completed for the actuation assembly 10 of the auto-injector of the present invention.

When the actuation sleeve 11 of the actuation assembly 10 of the auto-injector of the present invention is actuated to move relative to the execution sleeve 12 along the axis L direction, causing the first elastic element 13 to be gradually compressed, the actuation sleeve 11 will remove the restriction on the cantilever arms 121 of the execution sleeve 12, so that the cantilever arms 121 will bounce in the direction away from the axis L. At this time, the clamping part 1211 of the cantilever arms 121 will be separated from the groove 151 of the push rod 15. Therefore, the push rod 15 will be actuated by the compressed second elastic element 16 to move away from the rear cover 14.

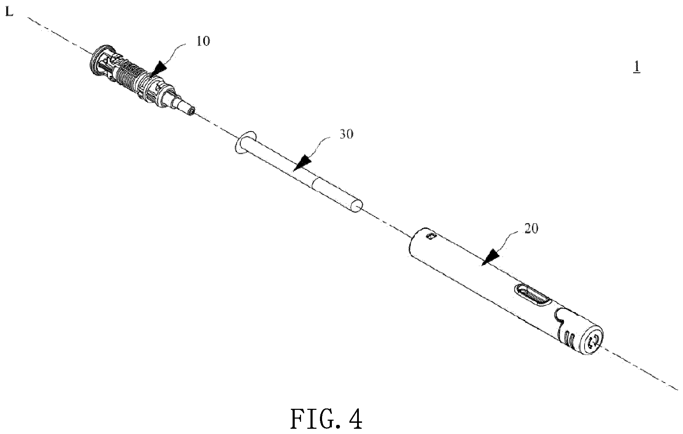

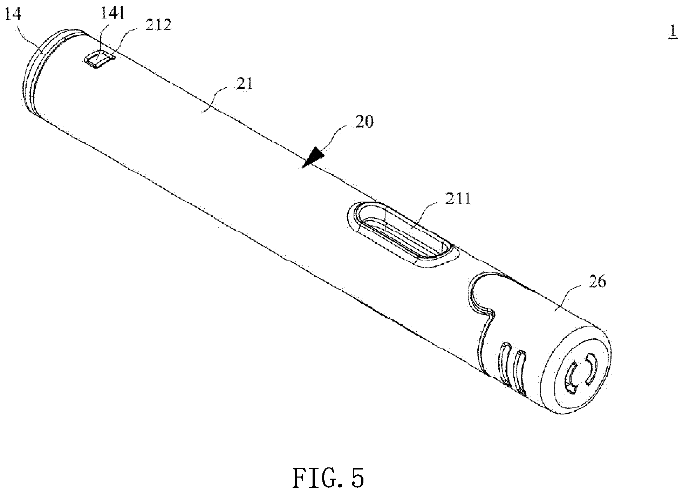

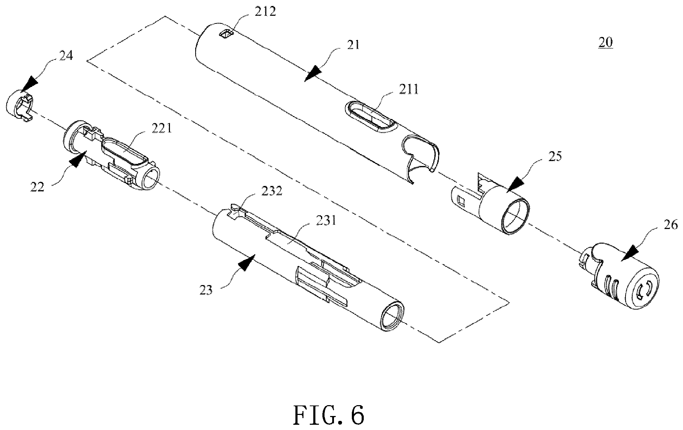

The auto-injector of the present invention is used for mounting and injecting a vial. Please refer to FIG.4 to 6, wherein FIG.4 is an exploded view of the auto-injector of the present invention, FIG.5 is a schematic diagram of the auto-injector of the present invention, and FIG. 6 is an exploded view of the accommodating assembly of the auto-injector of the present invention. As shown in FIG. 4 to 6, the auto-injector 1 of the present invention includes the aforementioned actuation assembly 10 and accommodating assembly 20. The accommodating assembly 20 is coupled to the actuation assembly 10 in one end that exposes the push rod 15, and the accommodating assembly 20 includes an outer housing 21, a vial sleeve 22 and a needle cap 23. The accommodating assembly 20 as a whole corresponds to axis L, so that the movable parts of the accommodating assembly 20 move relative to other parts along the direction of said axis L. The outer housing 21, vial sleeve 22 and the needle cap 23 can be made of the same or different solid materials, such as plastic material, metal or alloy, etc. In structural design, the outer housing 21, the vial sleeve 22 and the needle cap 23 are all similar in appearance to tubular parts, so that the hollow parts of the aforementioned parts can accommodate corresponding parts, but the invention is not limited thereto.

The outer housing 21 includes a plurality of buckle holes 211 and an inspection hole 212. A plurality of fastening holes 211 can be used for fastening the plurality of buckle elements 141 of the rear cover 14, so that the actuation assembly 10 is fixed to the outer housing 21 of the accommodating assembly 20 through the rear cover 14. Each of the aforementioned buckle elements 141 is provided with a protruding fastener structure on the outward side of the spring sheets, so that when the actuating component 10 is combined with the accommodating assembly 20 along the axis L direction, the fastener structure of each buckle element 141 is snapped into the buckle hole 211 through the action of the elastic arm, and then makes a sound due to the elastic force hitting the outer housing 21, so that the assembler can confirm the fastening between the plurality of buckle structures of the rear cover 14 and the plurality of buckle holes 211 of the outer housing 21. At this time, the rear cover 14 of the actuation assembly 10 can be fixed to the outer housing 21 of the accommodating assembly 20. The inspection hole 212 allows the user to confirm the injection state of the vial 30.

The vial sleeve 22 is coupled to the outer housing 21 for mounting the vial 30. The vial sleeve 22 includes an inspection part 221, which is disposed on the outer surface of the vial sleeve 22. Besides, the vial sleeve 22 protrudes from the outer surface to a sufficient height, so that the vial sleeve 22 is fixed on the inspection hole 212 of the outer housing 21 through the inspection part 221. In one embodiment of the present invention, the vial sleeve 22 at least for the inspection part 221 can be made of a transparent material, so as to facilitate the user to inspect the vial 30 through the inspection part 221.

The needle cap 23 threads through the outer housing 21, and a part of the needle cap 23 is exposed at one end of the outer housing 21. The needle cap 23 is movable relative to the outer housing 21 and the vial sleeve 22, and the actuation sleeve 11 can be actuated by movement of the needle cap 23. The needle cap 23 includes a sliding groove 231 and a plurality of corresponding joint parts 232. In one embodiment of the present invention, when the vial sleeve 22 is threaded into the needle cap 23, the inspection part 221 of the vial sleeve 22 passes through the sliding groove 231 and is fastened to the inspection hole 212 of the outer housing 21. And when the needle cap 23 moves relative to the vial sleeve 22, the inspection part 221 will move in the sliding groove 231. A plurality of corresponding joint parts 232 are disposed at one end of the needle cap 23 adjacent to the actuation assembly 10, for the needle cap 23 to move toward the actuation assembly 10 to induce the combination of the plurality of corresponding joint parts 114 and the plurality of corresponding joint parts 232 of the actuation sleeve 11, so that the needle cap 23 is linked together with the actuation sleeve 11. Wherein, a plurality of corresponding joint parts 232 are two open holes symmetrically arranged to match the plurality of joint parts 114.

In one embodiment of the present invention, the accommodating assembly 20 further includes a backing ring 24. The backing ring 24 is disposed at one end of the vial sleeve 22 adjacent to the actuation assembly 10, so that when the vial 30 is installed in the vial sleeve 22 of the accommodating assembly 20, the backing ring 24 can be used as the installation and injection buffer of the vial 30.

In addition, in the embodiment of the present invention, the accommodating assembly 20 also includes extension parts 25 and cap 26. The extension part 25 is disposed at the opposite end of the accommodating assembly 20 coupled to the actuation assembly 10, and the extension part 25 is fixed to the vial sleeve 22. The cap 26 is used to close the opposite end of the outer housing 21 of the accommodating assembly 20 combined with the actuation assembly 10, so that the auto-injector 1 of the present invention can be prevented from exposing to the needle cap 23 of the outer housing through the cap 26 when it is not in use.

During the assembly process of the auto-injector 1 of the present invention, the vial 30 is first installed in the vial sleeve 22 of the accommodating assembly 20. And at this time, the cap 26 of the accommodating assembly 20 closes one end of the exposed needle cap 23 of the outer housing 21. Then, the actuation assembly 10 is combined with the other end of the accommodating assembly 20. Accordingly, the assembly is completed for the auto-injector of the present invention.

As shown in FIG.1 to 6, the instruction is shown below on how to use auto-injector 1 of the present invention: the user may first remove the cap 26 from the outer housing 21 so that the needle cap 23 is exposed; then, press one end of the exposed needle cap 23 against the body part to be injected and apply force to the skin by grasping the outer housing so that the needle cap 23 moves inward toward the outer housing 21. At this time, the end of the needle cap 23 adjacent to the actuation assembly 10 will be pushed against the actuation sleeve 11, so that the actuation sleeve 11 is actuated to move relative to the actuation sleeve 12 along the axis L direction. In this way, the first elastic element 13 is gradually compressed. The moved actuation sleeve 11 will release the restriction on the cantilever arm 121 of the execution sleeve 12, so that the cantilever arm 121 pops out in the direction away from the axis L, causing the outward expanding portion 1212 of the cantilever arm 121 to strike the needle cap 23 and make a rattling sound, prompting the user that the injection procedure has been initiated, which is in compliance with the standards of medical regulations. Next, the clamping part 1211 of the cantilever arms 121 will be disengaged from the groove 151 of the push rod 15, so that the push rod 15 is actuated by the compressed second elastic element 16 to move toward the vial 30 in a non-disturbed state, thereby pushing the plunger of the vial 30 and driving the medicament toward the needle site. After the injection is completed, the user moves the auto-injector 1 of the present invention away from the body part. At this time, the actuation sleeve 11 returns to the original position by the elastic restoring force of the first elastic element 13, and drives the needle cap 23 to move and expose again on the outer housing 21 to conceal the needle site of the vial 30.

Although the embodiments of the present invention are disclosed as described above, they are not intended to limit the present invention. Any person skilled in the relevant art can make certain changes to the shape, structure, features, method and quantity described in the claims of the present invention without departing from the scope of the present invention. Thus, the scope of patent protection of the present invention shall be determined by the scope of the appended claims in this specification.

## Claims

1. An actuation assembly of an auto-injector, which is used for combining an accommodating assembly (20) with a vial (30), includes an actuation sleeve (11), an execution sleeve (12), a first elastic element (13), a rear cover (14), a push rod (15), and a second elastic element (16), the **characterized in that**:
the actuation sleeve (11), which includes a channel (111);
the execution sleeve (12), which is movably threaded through the channel (111) along an axis (L), the execution sleeve (12) includes two symmetrical cantilever arms (121), and each of the cantilever arms (121) includes a clamping part (1211);
the first elastic element (13), two ends of the first elastic element (13) abuts the actuation sleeve (11) and the execution sleeve (12) respectively;
the rear cover (14), coupled to one end of the execution sleeve (12);
the push rod (15), which is movably threaded through the execution sleeve (12) along the axis (L), the push rod (15) includes two grooves (151), when the cantilever arms (121) of the execution sleeve (12) is confined within the channel (111) of the actuation sleeve (11), the clamping part (1211) of the cantilever arms (121) is clipped into the groove (151) of the push rod (15) to restrict the movement of the push rod (15); and
the second elastic element (16), two ends of the second elastic element (16) abuts the rear cover (14) and the push rod (15) respectively, when the actuation sleeve (11) moves along the axis direction relative to the execution sleeve (12), the first elastic element (13) is gradually compressed, so that the clamping part (1211) of the cantilever arms (121) of the execution sleeve (12) is disengaged from the groove (151) of the push rod (15), and the push rod (15) is actuated toward the vial (30) by the second elastic element (16) which has been compressed.

2. The actuation assembly of the auto-injector of claim 1, wherein the actuation sleeve (11) includes a protruding part (112), which is disposed on the outer surface of the actuation sleeve (11), and the execution sleeve (12) further includes a plurality of abutting portions (122); both ends of the first elastic element (13) respectively abuts the protruding part (112) of the actuation sleeve (11) and the plurality of abutting portions (122) of the execution sleeve (12).

3. The actuation assembly of the auto-injector of claim 2, wherein the actuation sleeve (11) further comprises a plurality of grooves (113), and the plurality of abutting portions (122) of the execution sleeve (12) pass through the plurality of grooves (113) to protrude from the outer surface of the actuation sleeve (11), when the execution sleeve (12) moves relative to the actuation sleeve (11), the plurality of abutting portions (122) move along the plurality of grooves (113).

4. The actuation assembly of the auto-injector of claim 1, wherein the execution sleeve (12) further comprises a plurality of buckle parts (123), and the rear cover (14) further comprises a plurality of buckle elements (141) corresponding to a plurality of buckle parts (123).

5. The actuation assembly of the auto-injector of claim 1, wherein the rear cover (14) includes a plurality of guide elements (124), and the execution sleeve (12) further includes a plurality of corresponding guide elements (142); when the rear cover (14) moves relative to the execution sleeve (12), the rear cover (14) is moved relative to the execution sleeve (12) along the axis (L) with the cooperation between the guide elements (124) and the corresponding guide elements (142).

6. The actuation assembly of the auto-injector of claim 1, wherein the actuation sleeve (11) further comprises at least one stopper structure (116), which is protruded on the surface of the channel (111).

7. The actuation assembly of the auto-injector of claim 6, wherein each of the cantilevers arms (121) further comprises an outer expansion part (1212) corresponding to the at least one stopper structure (116).

8. The actuation assembly of the auto-injector of claim 6, wherein the actuation sleeve (11) further comprises at least one auxiliary mounting hole (115), which passes through the outer surface of the actuation sleeve (11) to the channel (111), and is located between one end of the actuation sleeve (11) adjacent to the rear cover (14) and the stopper structure (116).

9. An auto-injector, which is used for mounting and injecting a vial (30), includes the actuation assembly (10) according to claim 1 and an accommodating assembly (20), the accommodating assembly (20) includes an outer housing (21), a vial sleeve (22), and a needle cap (23), the **characterized in that**:
the accommodating assembly (20), coupled to one end of the actuation assembly (10);
the vial sleeve (22), which is coupled to the outer housing (21) for mounting the vial (30); and
the needle cap (23), which is threaded through and partially exposed to the outer housing (21) and can move relative to the outer housing (21) and the vial sleeve (22), when the needle cap (23) is moved toward the outer housing (21) by an external force, the needle cap (23) pushes against the actuation sleeve (11) of the actuation assembly (10), causing the clamping part (1211) of the cantilever arms (121) of the actuation sleeve (11) to disengage the groove (151) of the push rod (15), which in turn actuates the push rod (15) through the second elastic element (16) which has been compressed, thereby actuating the push rod (15) to move toward the vial (30) to complete the injection.

10. The auto-injector of claim 9, wherein the accommodating assembly (20) further comprises a backing ring (24), which is disposed at one end of the actuating assembly (10) adjacent to the vial sleeve (22).

## Patentansprüche

1. Eine Antriebsbaugruppe eines automatischen Injektors zum Verbinden der Anpassungsbaugruppe (20) mit einem Fläschchen (30), umfassend durch eine Antriebshülse (11), eine Ausführungshülse (12), ein erstes elastisches Element (13), eine Rückendeckung (14), eine Schubstange (15) und ein zweites elastisches Element (16), **gekennzeichnet durch**:
Die Antriebshülse (11), die einen Kanal (111) umfasst;
Die Ausführungshülse (12), die beweglich durch den Kanal (111) entlang der Achse (L) durchläuft, die Ausführungshülse (12) umfasst zwei symmetrische Auslegerarme (121), und jeder der Auslegerarme (121) umfasst einen Klemmteil (1211);
Das erste elastische Element (13), die beiden Enden vom ersten elastischen Element (13) sind getrennt nah an der Antriebshülse (11) und der Ausführungshülse (12);
Die Rückendeckung (14), gekoppelt mit einem Ende von der Ausführungshülse (12);
Die Schubstange (15), die beweglich durch die Ausführungshülse (12) entlang der Achse (L) durchläuft, die Schubstange (15) umfasst zwei Rillen (151), wenn die Auslegerarme (121) der Ausführungshülse (12) innerhalb des Kanals (111) der Antriebshülse (11) eingeschlossen ist, wird der Klemmteil (1211) der Auslegerarme (121) in die Rille (151) der Schubstange (15) geklemmt, um die Bewegung der Schubstange (15) zu beschränken; und
Das zweite elastische Element (16), die beiden Enden vom zweiten elastischen Element (16) sind getrennt nah an der Rückendeckung (14) und der Schubstange (15), wenn die Antriebshülse (11) sich entlang der Achsrichtung gegenüber Ausführungshülse (12) bewegt, wird das erste elastische Element (13) allmählich zusammengedrückt, so dass der Klemmteil (1211) der Auslegerarme (121) der Ausführungshülse (12) von der Rille (151) der Schubstange (15) getrennt ist, und die Schubstange (15) wird durch das zweite elastische Element (16), das zusammengedrückt wird, in Richtung vom Fläschchen (30) angetrieben.

2. Die Antriebsbaugruppe des automatischen Injektors nach Anspruch 1, wobei die Antriebshülse (11) auch einen erhabenen Teil (112) umfasst, der auf der Außenfläche der Antriebshülse (11) angeordnet wird, und die Ausführungshülse (12) umfasst auch eine Vielzahl von benachbarten Portionen (122); Die beiden Enden vom ersten elastischen Element (13) sind getrennt an den erhabenen Teil (112) der Antriebshülse (11) und der Vielzahl von benachbarten Portionen (122) der Ausführungshülse (12).

3. Die Antriebsbaugruppe des automatischen Injektors nach Anspruch 2, wobei die Antriebshülse (11) auch eine Vielzahl von Rillen (113) umfasst, und die Vielzahl von benachbarten Portionen (122) der Ausführungshülse (12) geht über die Vielzahl von Rillen (113), um aus der Außenfläche der Antriebshülse (11) zu herausragen, und wenn die Ausführungshülse (12) sich gegenüber Antriebshülse (11) bewegt, bewegt die Vielzahl von benachbarten Portionen (122) sich entlang der Vielzahl von Rillen (113).

4. Die Antriebsbaugruppe des automatischen Injektors nach Anspruch 1, wobei die Ausführungshülse (12) auch eine Vielzahl von Verschlussteilen (123), und die Rückendeckung (14) umfasst auch eine Vielzahl von Verschlussteilen (123), die einer Vielzahl von Verschlusselementen (141) entsprechen.

5. Die Antriebsbaugruppe des automatischen Injektors nach Anspruch 1, wobei die Rückendeckung (14) eine Vielzahl von Führungselementen (124), und die Ausführungshülse (12) umfasst auch eine Vielzahl von entsprechenden Führungselementen (142); Wenn die Rückendeckung (14) sich gegenüber Ausführungshülse (12) bewegt, bewegt sich die Rückendeckung (14) entlang der Achse (L) gegenüber Ausführungshülse (12) unter der Zusammenarbeit zwischen den Führungselementen (124) und den entsprechenden Führungselementen (142).

6. Die Antriebsbaugruppe des automatischen Injektors nach Anspruch 1, wobei die Antriebshülse (11) auch mindestens eine Stopperstruktur (116) umfasst, die aus der Oberfläche des Kanals (111) herausragt.

7. Die Antriebsbaugruppe des automatischen Injektors nach Anspruch 6, wobei jeder der Auslegerarme (121) auch ein externes Expansionsteil (1212) umfasst, das mindestens einer Stopperstruktur (116) entspricht.

8. Die Antriebsbaugruppe des automatischen Injektors nach Anspruch 6, wobei die Antriebshülse (11) auch mindestens eine Hilfsbefestigungsbohrung (115) umfasst, die durch die Außenfläche der Antriebshülse (11) zum Kanal (111) läuft und zwischen einem Ende der Antriebshülse (11) nah an der Rückendeckung (14) und der Stopperstruktur (116) angeordnet ist.

9. Der automatische Injektor zum Montieren und Injizieren von einem Fläschchen (30), umfasst nach Anspruch 1 eine Antriebsbaugruppe (10) und eine Anpassungsbaugruppe (20), und die Anpassungsbaugruppe (20) umfasst ein Gehäuse (21), eine Fläschchenhülse (22) und eine Nadelkappe (23), **gekennzeichnet durch**:
Die Anpassungsbaugruppe (20), gekoppelt mit einem Ende der Antriebsbaugruppe (10);
Die Fläschchenhülse (22), die mit dem Gehäuse (21) zum Befestigen vom Fläschchen (30) gekoppelt ist; und
Die Nadelkappe (23) durchläuft durch das Gehäuse (21) und enthüllt sich teilweise zum Gehäuse (21) und kann sich gegenüber Gehäuse (21) und Fläschchenhülse (22) bewegen, wenn die Nadelkappe (23) durch äußere Kraft zum Gehäuse (21) bewegt wird, erzeugt die Nadelkappe (23) eine Schubkraft auf die Antriebshülse (11) der Antriebsbaugruppe (10), so dass das Klemmteil (1211) der Auslegerarme (121) der Antriebshülse (11) von der Rille (151) der Schubstange (15) getrennt wird, was die Schubstange (15) nacheinander durch das zweite elastische Element (16) antreibt, das zusammengedrückt wird, und somit wird die Schubstange (15) angetrieben, sich in Richtung vom Fläschchen (30) zu bewegen, um die Injektion abzuschließen.

10. Der automatische Injektor nach Anspruch 9, wobei die Anpassungsbaugruppe (20) auch einen Stützring (24) umfasst, der an einem Ende der Antriebsbaugruppe (10) nah an der Fläschchenhülse (22) angeordnet ist.

## Revendications

1. Ensemble d'actionnement d'un auto-injecteur, destiné à être combiné à un ensemble de réception (20) équipé d'un flacon (30), comprenant un manchon d'actionnement (11), un manchon d'exécution (12), un premier élément élastique (13), un couvercle arrière (14), une tige de propulsion (15) et un deuxième élément élastique (16), **caractérisé en ce que** :
le manchon d'actionnement (11) comprend un canal (111) ;
le manchon d'exécution (12), qui traverse de manière mobile en filetage le canal (111) selon un axe (L), le manchon d'exécution (12) comprend deux bras en porte-à-faux (121) symétriques, chacun des bras en porte-à-faux (121) comportant une partie de serrage (1211) ;
le premier élément élastique (13), dont les deux extrémités du premier élément élastique (13) butent respectivement contre le manchon d'actionnement (11) et le manchon d'exécution (12) ;
le couvercle arrière (14), accouplé à une extrémité du manchon d'exécution (12) ;
la tige de propulsion (15), qui traverse de manière mobile en filetage le manchon d'exécution (12) selon l'axe (L), la tige de propulsion (15) comprend deux rainures (151) ; lorsque les bras en porte-à-faux (121) du manchon d'exécution (12) sont confinés dans le canal (111) du manchon d'actionnement (11), la partie de serrage (1211) des bras en porte-à-faux (121) est encliquetée dans la rainure (151) de la tige de propulsion (15) pour restreindre le mouvement de la tige de propulsion (15) ; et
le deuxième élément élastique (16), dont les deux extrémités du deuxième élément élastique (16) butent respectivement contre le couvercle arrière (14) et la tige de propulsion (15) ; lorsque le manchon d'actionnement (11) se déplace selon la direction de l'axe par rapport au manchon d'exécution (12), le premier élément élastique (13) est progressivement comprimé, de sorte que la partie de serrage (1211) des bras en porte-à-faux (121) du manchon d'exécution (12) se détache de la rainure (151) de la tige de propulsion (15), et la tige de propulsion (15) est actionnée vers le flacon (30) par le deuxième élément élastique (16) qui a été comprimé.

2. Ensemble d'actionnement de l'auto-injecteur selon la revendication 1, **caractérisé en ce que** le manchon d'actionnement (11) comprend une partie en saillie (112) disposée sur la surface extérieure du manchon d'actionnement (11), et le manchon d'exécution (12) comprend en outre une pluralité de parties d'appui (122) ; les deux extrémités du premier élément élastique (13) butent respectivement contre la partie en saillie (112) du manchon d'actionnement (11) et la pluralité de parties d'appui (122) du manchon d'exécution (12).

3. Ensemble d'actionnement de l'auto-injecteur selon la revendication 2, **caractérisé en ce que** le manchon d'actionnement (11) comprend en outre une pluralité de rainures (113), et la pluralité de parties d'appui (122) du manchon d'exécution (12) traversent la pluralité de rainures (113) pour dépasser de la surface extérieure du manchon d'actionnement (11) ; lorsque le manchon d'exécution (12) se déplace par rapport au manchon d'actionnement (11), la pluralité de parties d'appui (122) se déplacent le long de la pluralité de rainures (113).

4. Ensemble d'actionnement de l'auto-injecteur selon la revendication 1, **caractérisé en ce que** le manchon d'exécution (12) comprend en outre une pluralité de parties à crans (123), et le couvercle arrière (14) comprend en outre une pluralité d'éléments à crans (141) correspondant à la pluralité de parties à crans (123).

5. Ensemble d'actionnement de l'auto-injecteur selon la revendication 1, **caractérisé en ce que** le couvercle arrière (14) comprend une pluralité d'éléments de guidage (124), et le manchon d'exécution (12) comprend en outre une pluralité d'éléments de guidage correspondants (142) ; lorsque le couvercle arrière (14) se déplace par rapport au manchon d'exécution (12), le couvercle arrière (14) se déplace par rapport au manchon d'exécution (12) selon l'axe (L) grâce à la coopération entre les éléments de guidage (124) et les éléments de guidage correspondants (142).

6. Ensemble d'actionnement de l'auto-injecteur selon la revendication 1, **caractérisé en ce que** le manchon d'actionnement (11) comprend en outre au moins une structure d'arrêt (116) qui fait saillie sur la surface du canal (111).

7. Ensemble d'actionnement de l'auto-injecteur selon la revendication 6, **caractérisé en ce que** chacun des bras en porte-à-faux (121) comprend en outre une partie d'expansion externe (1212) correspondant à l'au moins une structure d'arrêt (116).

8. Ensemble d'actionnement de l'auto-injecteur selon la revendication 6, **caractérisé en ce que** le manchon d'actionnement (11) comprend en outre au moins un trou auxiliaire de montage (115) qui traverse la surface extérieure du manchon d'actionnement (11) jusqu'au canal (111), et est situé entre une extrémité du manchon d'actionnement (11) adjacente au couvercle arrière (14) et la structure d'arrêt (116).

9. Auto-injecteur, destiné à monter et injecter un flacon (30), comprenant l'ensemble d'actionnement (10) selon la revendication 1 et un ensemble de réception (20), l'ensemble de réception (20) comprenant un boîtier externe (21), un manchon de flacon (22) et un capuchon d'aiguille (23), **caractérisé en ce que** :
l'ensemble de réception (20) est accouplé à une extrémité de l'ensemble d'actionnement (10) ;
le manchon de flacon (22), accouplé au boîtier externe (21) pour monter le flacon (30) ; et
le capuchon d'aiguille (23), qui traverse et est partiellement exposé au boîtier externe (21) et peut se déplacer par rapport au boîtier externe (21) et au manchon de flacon (22) ; lorsque le capuchon d'aiguille (23) est déplacé vers le boîtier externe (21) par une force externe, le capuchon d'aiguille (23) pousse contre le manchon d'actionnement (11) de l'ensemble d'actionnement (10), amenant la partie de serrage (1211) des bras en porte-à-faux (121) du manchon d'actionnement (11) à se détacher de la rainure (151) de la tige de propulsion (15), ce qui à son tour actionne la tige de propulsion (15) par le biais du deuxième élément élastique (16) qui a été comprimé, actionnant ainsi la tige de propulsion (15) pour se déplacer vers le flacon (30) afin de compléter l'injection.

10. Auto-injecteur selon la revendication 9, **caractérisé en ce que** l'ensemble de réception (20) comprend en outre une bague de support (24) disposée à une extrémité de l'ensemble d'actionnement (10) adjacente au manchon de flacon (22).
